# EUROPÄISCHE PATENTSCHRIFT

(11) **EP 2 306 900 B1**
(45) Veröffentlichungstag und Bekanntmachung des Hinweises auf die Patenterteilung: **05.10.2016**
(21) Anmeldenummer: 09757462.8
(22) Anmeldetag: 29.05.2009
(51) Int. Cl.: A61B 5/15

(54) **STECHVORRICHTUNG FÜR EINE BLUTPROBENENTNAHME UND VERFAHREN ZUM ENTNEHMEN EINER BLUTPROBE**
PRICKING DEVICE AND METHOD FOR TAKING A BLOOD SAMPLE
DISPOSITIF DE PIQÛRE ET PROCÉDÉ PERMETTANT LE PRÉLÈVEMENT D'UN ÉCHANTILLON DE SANG

(30) Priorität: 06.06.2008 DE 102008027267; 21.08.2008 DE 102008039111
(43) Veröffentlichungstag der Anmeldung: 13.04.2011
(73) Patentinhaber: Gerresheimer Regensburg GmbH, 93051 Regensburg (DE)
(72) Erfinder: BUTZ, Marion, 93049 Regensburg (DE); KNIE, Andreas, 93051 Regensburg (DE)
(74) Vertreter: Hannke, Christian
(86) Internationale Anmeldenummer: PCT/EP2009/056594
(87) Internationale Veröffentlichungsnummer: WO 2009/147080

(56) Entgegenhaltungen:
- EP-A- 1 090 584
- EP-A- 1 669 028
- US-A- 4 643 189
- US-A- 5 951 582
- US-A1- 2005 145 520

## Beschreibung

Die Ausführung betrifft einerseits eine Stechvorrichtung für eine Blutprobenentnahme mit einer verlagerbaren Halteeinrichtung für ein Stechmittel, mit einer Linearführung zum Führen der verlagerbaren Halteeinrichtung, mit Mitteln zum Antreiben der verlagerbaren Halteeinrichtung und mit einer Auslöseeinrichtung zum Auslösen einer Stechbewegung des Stechmittels, bei welcher nach einem manuellen Betätigen der Auslöseeinrichtung die verlagerbare Halteeinrichtung mittels der Antriebsmittel translatorisch verlagert werden kann. Andererseits betrifft es ein Verfahren zum Entnehmen einer Blutprobe, bei welchem ein Stechmittel vorwärts und anschließend direkt rückwärts bewegt wird, wodurch das Stechmittel zumindest mit seiner Spitze kurzzeitig in einen Körper eindringen kann, bei welchem das Stechmittel in einer verlagerbaren Halteeinrichtung translatorisch geführt wird, und bei welchem die verlagerbare Halteeinrichtung von einem Federantrieb angetrieben wird.

Gattungsgemäße Vorrichtungen sind aus dem Stand der Technik gut bekannt. Sie werden bevorzugt zum Entnehmen eines Bluttropfens verwendet, um anhand des Bluttropfens eine medizinische Untersuchung, beispielsweise eine Blutzuckermessung, vornehmen zu können. Hierzu kann eine derartige Vorrichtung auf einen Hautbereich eines Patienten aufgesetzt werden, um ein Einstechen eines geeigneten Stechmittels, wie etwa eine auswechselbare Lanzette der Vorrichtung, in die Haut zu ermöglichen, wobei nach dem Einstechen des Stechmittels aus verletzten Kapillargefäßen in oberen Hautschichten ein oder mehrere Bluttropfen entnommen werden können. Um Verletzungen an den Hautschichten jedoch möglichst gering zu halten, werden solche Stechmittel oftmals entlang einer Stechachse translatorisch vor und zurück bewegt. Beispielsweise ist aus der Patentschrift US 5,527,334 eine gattungsgemäße Stechhilfe beschrieben, bei welcher eine Nadel bzw. eine Lanzette von einem Nadelhalter gehalten wird, wobei der Nadelhalter zwischen Führungsrippen linear beweglich innerhalb der Stechhilfe gelagert ist. Angetrieben wird die Nadel von einer spannbaren Torsionsfeder, welche einerseits mit einem ersten Schenkelende in einem Gehäuse der Stechhilfe und andererseits mit einem zweiten Schenkelende in einem beweglichen Kupplungsteil der Stechhilfe befestigt ist. Das Kupplungsteil ist mit einem ersten Endbereich formschlüssig aber elastisch mit dem Nadelhalter verbunden, mit einem anderen Endbereich ist es in einem Führungsschlitz gelagert. Entspannt sich die Torsionsfeder durch ein manuelles Betätigen einer entsprechenden Auslöseeinrichtung, kann der andere Endbereich des Kupplungsteils entlang des Führungsschlitzes verlagert werden, wodurch das Kupplungsteil den Nadelhalter linear vor und zurück bewegen kann. Hierdurch wiederum kann die Nadel eine Stechbewegung ausführen, bei welcher die Nadelspitze kurzzeitig aus dem Gehäuse der Stechhilfe heraus schnellen kann.

Aus der EP 1090584 A2 ist eine Blutlanzettenvorrichtung zur Entnahme von Blut für Diagnosezwecke offenbart. Die hierin offenbarte Vorrichtung soll ein präzises und vibrationsarmes Einstichverhalten mit reduziertem Herstellungsaufwand aufweisen. Diese Vorrichtung weist ein Gehäuse, eine Lanzette und einen Lanzettenantrieb auf.

Die EP 1 669 028 A1 zeigt eine Lanzettenvorrichtung zum Erzeugen einer Einstichwunde sowie eine Lanzettenantriebs-Baugruppe. Die Lanzettenvorrichtung umfasst hierbei ein Gehäuse, einen Lanzettenantrieb, einen Lanzetten-Kopplungsmechanismus, ein Referenzelement, welches relativ zu dem Gehäuse bewegbar ist sowie einen Referenzelement-Kopplungsmechanismus.

In der US 2005/0145520 A1 ist unteranderem eine Stechhilfenvorrichtung beschrieben, welcher über eine Kartusche Punktionsnadeln zugeführt werden. Hierfür ist in der Stechhilfe eine Aufnahme vorgesehen, welche dazu geeignet ist, eine Nadel aus der Kartusche zu entnehmen und festzuhalten, um einen Stechvorgang auszuführen.

Es ist Aufgabe der Erfindung gattungsgemäße Stechvorrichtungen, häufig auch als Stechhilfen bezeichnet, für eine Blutprobenentnahme weiterzuentwickeln.

Die Aufgabe der Erfindung wird wie in Anspruch 1 beschreiben, von einer Stechvorrichtung für eine Blutprobenentnahme mit einer verlagerbaren Halteeinrichtung für ein Stechmittel, mit einer Linearführung zum Führen der verlagerbaren Halteeinrichtung, mit Mitteln zum Antreiben der verlagerbaren Halteeinrichtung und mit einer Auslöseeinrichtung zum Auslösen einer Stechbewegung des Stechmittels gelöst, bei welcher nach einem manuellen Betätigen der Auslöseeinrichtung die verlagerbare Halteeinrichtung mittels der Antriebsmittel translatorisch verlagert werden kann, wobei sich die Stechvorrichtung dadurch auszeichnet, dass die Antriebsmittel eine Kurvenbahn aufweisen, in welcher die verlagerbare Halteeinrichtung unmittelbar eingreift.

Greift die Halteeinrichtung unmittelbar selbst in eine Kurvenbahn der Antriebsmittel ein, kann der konstruktive Aufbau der Stechvorrichtung sehr einfach gehalten werden. So kann die Stechvorrichtung auch besonders flach bauen. Insbesondere können auf zusätzliche Kupplungseinrichtungen zwischen der Halteeinrichtung und der Antriebsmittel verzichtet werden, so dass geringere Massen während der Stechbewegung des Stechmittels bzw. der Halteeinrichtung beschleunigt und/oder verzögert werden müssen. Hierdurch wiederum kann eine Stechbewegung wesentlich schneller durchgeführt werden, wodurch ein Einstichschmerz verringert werden kann. Vorteilhafter Weise ist die Halteeinrichtung mittels der Kurvenbahn translatorisch angetrieben, so dass eine translatorische Stechbewegung, etwa einer Lanzette, mittels der Kurvenbahn bewerkstelligt werden kann. Die Kurvenbahn dient hierbei als Leitkurve der vorliegenden Halteeinrichtung.

Als Stechmittel kommen jegliche Gebilde, wie etwa Lanzetten bzw. Nadeln, in Frage, welche in geeigneter Weise leicht in obere Hautschichten eindringen können. Hierbei kann ein Stechmittel beispielsweise nur entlang einer geraden Bahn vor und zurück und/oder entlang einer Bahnkurve bewegt werden.

Es versteht sich, dass ein Verlauf der hier vorliegenden Kurvenbahn vielfältig gewählt und gestaltet sein kann, um die Halteeinrichtung vorteilhaft zu verlagern. Besonders einfach gestaltet sich ein Bewegungsablauf hinsichtlich der Kurvenbahn, wenn die Kurvenbahn in sich geschlossen ausgebildet ist. Demzufolge handelt es sich erfindungsgemäß bei der Kurvenbahn um eine Endlos-Kurvenbahn. Insbesondere können die im Zusammenhang mit der Kurvenbahn erläuterten Vorteile auch auf die Endlos-Kurvenbahn übertragen werden. Speziell bei einer Endlos-Kurvenbahn können die Antriebsmittel eine besonders gleichförmige Antriebsbewegung durchlaufen, wodurch ein Einstichergebnis des Stichmittels hinsichtlich eines Körpers, wie einer oberen Hautschicht, wesentlich verbessert werden kann.

In diesem Zusammenhang wird die Aufgabe der Erfindung auch von einem Verfahren wie in Anspruch 10 zum Entnehmen einer Blutprobe gelöst, bei welchem ein Stechmittel vorwärts und anschließend direkt rückwärts bewegt wird, wodurch das Stechmittel zumindest mit seiner Spitze kurzzeitig in einen Körper eindringen kann, bei welchem das Stechmittel in einer verlagerbaren Halteeinrichtung translatorisch geführt wird, und bei welchem die verlagerbare Halteeinrichtung von einem Federantrieb angetrieben wird, wobei die verlagerbare Halteeinrichtung unmittelbar innerhalb und entlang einer Endlos-Kurvenbahn eines Steuerkurvenbahnelementes geführt wird.

Vorteilhafter Weise bewegt sich die Halteeinrichtung bei einer derartigen in sich geschlossenen Endlos-Kurvenbahn lediglich in eine einzige Umlaufrichtung, wodurch die gesamte Bewegungskinematik bewegbarer Bauteile innerhalb der Stechvorrichtung vereinfacht werden kann. Insbesondere kann auf Grund einer derartigen Bewegungskinematik ein Nachschwingen des Stechmittels bei der Stechbewegung verringert bzw. idealerweise sogar zur Gänze ausgeschlossen werden, wodurch die Gefahr eines Nachstechens des Stechmittels weiter verringert werden kann. Hierdurch kann ein Stechvorgang zudem wesentlich schmerzarmer erfolgen. Insofern kann eine Blutprobenentnahme für einen Patienten wesentlich angenehmer durchgeführt werden.

Der Federantrieb kann vorliegend problemlos und sehr kompakt bereitgestellt werden, wenn die Antriebsmittel ein Federelement zum Speichern von Energie umfassen.

Vorzugsweise kann das Federelement als Schenkelfeder relativ flach konstruiert werden kann.

Weiter sieht eine vorteilhafte Verfahrensvariante auch vor, dass die Halteeinrichtung sowohl bei einer Vorwärtsbewegung des Stechmittels als auch bei einer Rückwärtsbewegung des Stechmittels immer nur in eine einzige Umlaufrichtung entlang der Kurvenbahn bewegt wird. Insbesondere hierdurch kann die Gefahr eines Nachschwingens der verlagerbaren Halteeinrichtung außergewöhnlich wirkungsvoll verringert werden.

Eine diesbezüglich bevorzugte Ausführungsvariante sieht somit auch vor, dass die Antriebsmittel ein Steuerkurvenbahnelement umfassen, welches die Endlos-Kurvenbahn aufweist. Hierdurch kann die Endlos-Kurvenbahn besonders gut geführt werden.

Ist das Steuerkurvenbahnelement der Antriebsmittel um eine Schwenkachse schwenkbar gelagert, kann die Halteeinrichtung problemlos translatorisch an der Linearführung translatorisch verlagerbar gelagert sein.

Deshalb ist auch hinsichtlich einer weiteren vorteilhaften Verfahrensvariante vorgesehen, dass die Kurvenbahn entlang eines Bogenabschnittes um eine Schwenkachse geschwenkt wird, wodurch die verlagerbare Halteeinrichtung translatorisch bewegt wird. Eine diesbezügliche Schwenkbewegung kann mittels einer Schenkelfeder vorteilhaft eingeleitet werden.

Ist zudem das Federelement an einer Schwenkachse eines Steuerkurvenbahnelementes gelagert, kann der konstruktive Aufbau der vorliegenden Stechvorrichtung weiter vereinfacht werden.

Vorzugsweise ist die Kurvenbahn oval ausgebildet, wodurch ein Beschleunigungs- und Geschwindigkeitsprofil hinsichtlich der Halteeinrichtung des Stechmittels zusätzlich beeinflussbar ist. Insbesondere kann die Halteeinrichtung in gerader ausgebildeten Bereichen einer ovalen Kurvenbahn wesentlich besser beschleunigt werden, so dass hierdurch insbesondere eine schnellere Vorwärtsbewegung des Stechmittels bzw. der Halteeinrichtung erzielt werden kann. Vermutlich liegt es auch daran, dass in den gerader ausgebildeten Bereichen der ovalen Kurvenbahn eine geringere Reibung zwischen der Halteeinrichtung und Führungsbereichen der Kurvenbahn vorliegt.

Erfindungsgemäß weist die Kurvenbahn eine Kulisse auf, innerhalb welcher die Halteeinrichtung zumindest teilweise angeordnet und geführt ist. Weist die Kurvenbahn eine Kulisse auf, beispielsweise in Gestalt einer Nut, kann die Halteeinrichtung besonders betriebssicher entlang der Kurvenbahn geführt werden.

Die Halteeinrichtung kann baulich besonders einfach in die Kurvenbahn, insbesondere in die Kulisse, eingreifen, wenn die Halteeinrichtung einen Kurvenfolger aufweist. Ein solcher Kurvenfolger kann sehr einfach mittels eines Führungszapfens realisiert sein, der in die Kulisse eingreift. Vorzugsweise ist der Führungszapfen von der Halteeinrichtung selbst gebildet. Beispielsweise wird ein Kurvenfolger hierzu in einem Spritzgießverfahren an die Halteeinrichtung angespritzt. Es versteht sich, dass der Kurvenfolger aber auch auf andere Weise mit der Halteeinrichtung verbunden sein kann.

Ist der Kurvenfolger der Halteeinrichtung sowohl bei einer translatorischen Vorwärtsbewegung der Halteeinrichtung als auch bei einer translatorischen Rückwärtsbewegung der Halteeinrichtung immer nur in eine Umlaufrichtung entlang der Kurvenbahn bewegbar, kann lediglich mittels der vorliegenden Halteeinrichtung eine im Wesentlichen kreisförmige Antriebsbewegung unmittelbar in eine lineare Abtriebsbewegung überführt werden.

Hervorragend kann eine solche Bewegungsüberführung erzielt werden, wenn ein Kurvenfolger der Halteeinrichtung innerhalb einer Kulisse einer Kurvenbahn derart angeordnet ist, dass der Kurvenfolger innerhalb der Kulisse nur in eine einzige Umlaufrichtung entlang der Kurvenbahn bewegbar gelagert ist. Zudem kann eine Bewegungskinematik des Stechmittels an einer Stechvorrichtung vorteilhaft ausgeführt werden.

Insbesondere eine gute Auslösesicherheit der vorliegenden Stechvorrichtung kann gewährleistet werden, wenn der Kurvenfolger der Halteeinrichtung innerhalb der Kurvenbahn in einer Startposition angeordnet ist, und die Kurvenbahn hinsichtlich der Startposition einen Einlaufbereich für den Kurvenfolger und einen Auslaufbereich für den Kurvenfolger aufweist, wobei der Einlaufbereich und der Auslaufbereich voneinander verschieden sind. Befindet sich der Kurvenfolger in einem betriebsbereiten Zustand der Stechvorrichtung derart in einer Startposition, dass der Kurvenfolger beim Auslösen der Stechvorrichtung über den Auslaufbereich aus der Startposition heraus und nach Ausführen einer Stechbewegung des Stechmittels über den Einlaufbereich erneut in die Startposition hinein gelangen kann, kann die Betriebssicherheit der Stechvorrichtung weiter verbessert werden.

Weitere Vorteile, Ziele und Eigenschaften vorliegender Erfindung werden anhand nachfolgender Beschreibung anliegender Zeichnung erläutert, in welcher beispielhaft eine mehrmals betätigbare Stechvorrichtung zum Entnehmen einer Blutprobe mit einem Stechmittel dargestellt ist.

Es zeigt
- Figur 1: schematisch eine erste geschnittene Ansicht einer Stechvorrichtung zum Entnehmen einer Blutprobe in einem betriebsbereiten Zustand, in welchem ein Kurvenfolger einer Halteeinrichtung in einer Startposition innerhalb einer Kurvenbahn eines Steuerkurvenbahnelementes angeordnet ist;
- Figur 2: schematisch eine weitere geschnittene Ansicht der Stechvorrichtung in dem betriebsbereiten Zustand der Figur 1;
- Figur 3: schematisch eine perspektivische geschnittene Ansicht der Stechvorrichtung in dem betriebsbereiten Zustand der Figuren 1 und 2;
- Figur 4: schematisch eine geschnittene Ansicht der Stechvorrichtung in einer Stechposition eines Stechmittels der Stechvorrichtung;
- Figur 5: schematisch eine weitere geschnittene Ansicht der Stechvorrichtung in der Stechposition der Figur 4;
- Figur 6: schematisch eine geschnittene Ansicht der Stechvorrichtung in einer Ruheposition nach einer erfolgten Stechbewegung und in einem ungespannten Zustand;
- Figur 7: schematisch eine geschnittene Ansicht der Stechvorrichtung während eines Spannvorgangs mit einem erneut gespannten Federantrieb; und
- Figur 8: schematisch eine perspektivische Ansicht des Gehäuses der Stechvorrichtung aus den Figuren 1 bis 7.

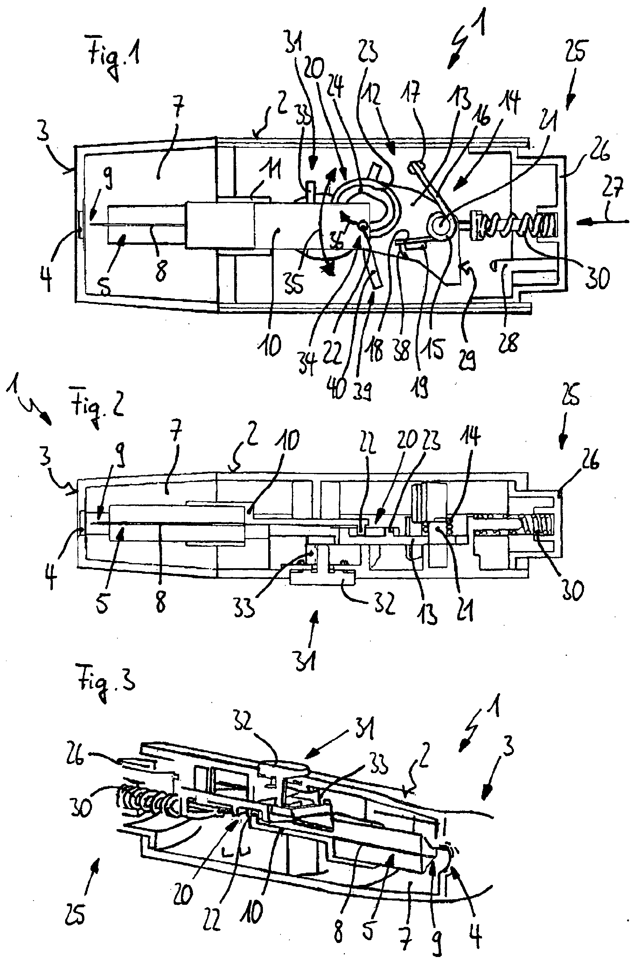

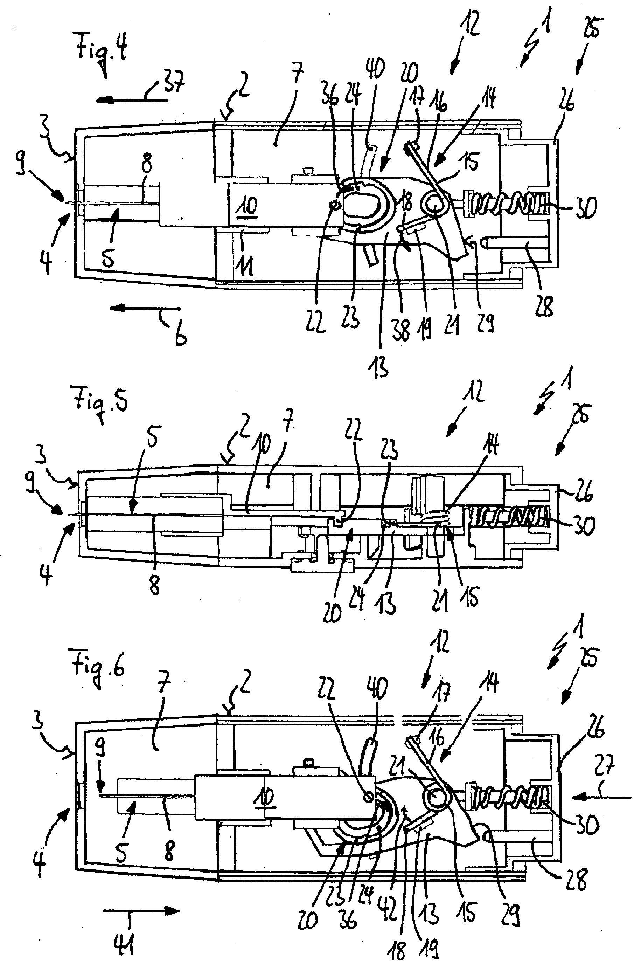

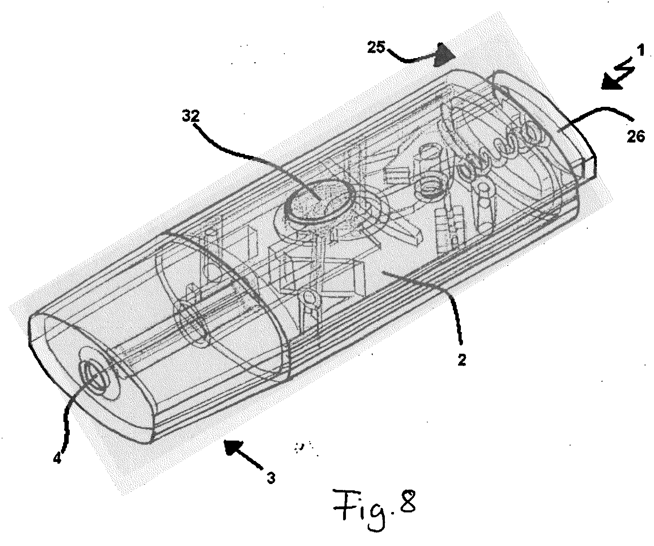

Die in den Figuren 1 bis 8 gezeigte Stechvorrichtung 1 für eine Blutprobenentnahme weist ein flaches Gehäuse 2 (siehe insbesondere Figur 8) auf, an welchem unterseitig 3 eine Behandlungsöffnung 4 vorgesehen ist, durch welche hindurch zur Behandlung ein Stechmittel 5 gemäß einer Stechbewegung 6 (siehe Figuren 4 und 5) kurzzeitig aus einem Gehäuseinneren 7 der Stechvorrichtung 1 hervorschnellen kann. Bei der Stechvorrichtung 1 handelt es sich um eine mehrmals betätigbare Stechvorrichtung 1, die es ermöglicht, dass das Stechmittel 5 nach einem erfolgten Einstich sofort wieder in das Gehäuse 2 zurückgezogen und anschließend die Stechvorrichtung 1 wieder vorgespannt werden kann, um ein weiteres Mal betätigt werden zu können.

Das Stechmittel 5 in Gestalt einer Stechnadel 8 wird nach einem erfolgten Einstich in eine obere Hautschicht eines Patienten sofort wieder in das flache Gehäuse 2 zurückgezogen, so dass von der Stechnadelspitze 9 der Stechnadel 8 keine weitere Verletzungsgefahr ausgehen kann.

Das Stechmittel 5 ist mit einer geeigneten verlagerbaren Halteeinrichtung 10, oftmals auch als Lancet-Holder bezeichnet, gehalten, wobei die verlagerbare Halteeinrichtung 10 mittels einer Linearführung 11 translatorisch entlang einer fiktiven Stechachse, welche durch die Stechnadel 8 verkörpert wird, vor und zurück bewegt werden kann.

Damit das Stechmittel 5 idealerweise immer eine nahezu identische Stechbewegung 6 durchlaufen kann, weist die Stechvorrichtung 1 des Weiteren Mittel 12 zum Antreiben der verlagerbaren Halteeinrichtung 10 auf.

Diese Antriebsmittel 12 umfassen in diesem Ausführungsbeispiel insbesondere ein Steuerkurvenbahnelement 13 und ein Federelement 14 in Gestalt einer Schenkelfeder 15. Das Federelement 14 ist hierbei mit einem ersten Schenkel 16 in einer Gehäusehalterung 17 und mit einem zweiten Schenkel 18 in einer Halterung 19 des Steuerkurvenbahnelementes 13 eingeklemmt, so dass es gespannt werden kann.

Die Antriebsmittel 12 stellen darüber hinaus eine Kurvenbahn 20 bereit, in welcher die verlagerbare Halteeinrichtung 10 unmittelbar eingreift. Die Kurvenbahn 20 ist hierbei dem Steuerkurvenbahnelement 13 zugeordnet. Damit die Halteeinrichtung 10 betriebssicher der Kurvenbahn 20 folgen kann, wenn das Steuerkurvenbahnelement 13 um eine Schwenkachse 21 verschwenkt wird, bildet die Halteeinrichtung 10 einen Kurvenfolger 22 aus, der in einer Kulisse 23 der Kurvenbahn 20 angeordnet ist. Die Kurvenbahn 20 an sich ist an dem Steuerkurvenbahnelement 13 als eine ovale Endlos-Kurvenbahn 24 ausgebildet, so dass der Kurvenfolger 22 auch endlos der Kurvenbahn 20 in nur einer Richtung folgen kann.

Um das Steuerkurvenbahnelement 13 manuell derart verlagern zu können, dass das Federelement 14 mit einer ausreichend großen Federkraft vorgespannt werden kann, ist oberseitig 25 des flachen Gehäuses 2 ein handbetätigbares Tastenelement 26 vorgesehen, welches gemäß der Pfeilrichtung 27 in das flache Gehäuse 2 hinein gedrückt werden kann. Während einer solchen Betätigung kann das Steuerkurvenbahnelement 13 verlagert, das Federelement 14 vorgespannt und die gesamte Stechvorrichtung 1 in einen gespannten und betriebsbereiten Zustand, wie er in Figur 1 gezeigt ist, überführt werden. Während des Spannvorgangs kann ein Tastenelementsteg 28 des handbetätigbaren Tastenelementes 26 gegen einen Vorsprung 29 des Steuerkurvenbahnelementes 13 drücken, wodurch das Steuerkurvenbahnelement 13 um die Schwenkachse 21 geschwenkt wird. Hierdurch wird das Federelement 14 gespannt. Das Tastenelement 26 kann anschließend mittels einer Spiralfeder 30 wieder in seine Ausgangslage (siehe Figur 1) zurückbewegt werden.

Um die Stechvorrichtung 1 auslösen zu können, weist die Stechvorrichtung 1 eine geeignete Auslöseeinrichtung 31 auf, die einen Drücker 32 (siehe beispielsweise Figuren 2 und 3) mit einem Rastarm 33 umfasst. Der Rastarm 33 kann in geeigneter Weise mit der verlagerbaren Halteeinrichtung 10 korrespondieren, so dass die verlagerbare Halteeinrichtung 10 insbesondere bei vorgespanntem Federelement 14 mit dem Rastarm 33 verrastet ist. Hierdurch kann solange eine Stechbewegung 6 verhindert werden, wie der Drücker 32 der Auslöseeinrichtung 31 nicht betätigt und die Auslöseeinrichtung 31 nicht ausgelöst wird.

Gemäß den Darstellungen der Figuren 1 bis 3 befindet sich die Stechvorrichtung 1 in einem gespannten und betriebsbereiten Zustand, in welchem sich der Kurvenfolger 22 innerhalb der Kulisse 23 in einer Startposition 34 auf der Kurvenbahn 20 befindet. Die Geometrien und das Zusammenspiel des Steuerkurvenbahnelementes 13, der Kurvenbahn 20, eines Schwenkvermögens 35 des Steuerkurvenelementes 13 und damit auch der Kurvenbahn 20, der Halteeinrichtung 10 sowie des Kurvenfolgers 22 sind derart gewählt, dass der Kurvenfolger 22 sich in nur eine Umlaufrichtung 36 entlang der Kurvenbahn 20 bewegen kann. Insbesondere hierdurch kann gewährleistet werden, dass die Halteeinrichtung 10 mit dem Stechmittel 5 nach der eigentlichen Stechbewegung 6 nicht weiter nachschwingen kann, so dass sichergestellt ist, dass pro Betätigung der Stechvorrichtung 1 das Stechmittel 5 nur ein einziges Mal durch die Behandlungsöffnung 4 gelangen kann.

Zum Auslösen der betriebsbereiten Stechvorrichtung 1 wird der Drücker 32 manuell betätigt, wodurch der Rastarm 33 die Steuerkurvenbahnelemente 13 frei gibt. Die Halteeinrichtung 10 kann dann eine translatorische Vorwärtsbewegung 37 (siehe Figur 4) im Sinne der Stechbewegung 6 durchlaufen, wobei die translatorische Vorwärtsbewegung 37 und eine damit verbundene mögliche Druckbeaufschlagung des Stechmittels 5 durch die Federkraft bzw. eine Federbewegung 38 der auseinanderdrückenden Schenkelfeder 15 bzw. der Federbewegung 38 des zweiten Schenkels 18 der Schenkelfeder 15 initiiert wird. Durch die Federbewegung 38 wird das Steuerkurvenbahnelement 13 um die Schwenkachse 21 geschwenkt. Hierbei wird das Steuerkurvenbahnelement 13 zusätzlich in einer Führung 39 entlang eines Bogenabschnittes 40 geführt.

Durch die Schwenkbewegung des Steuerkurvenbahnelementes 13 folgt der Kurvenfolger 22 der Kurvenbahn 20 gemäß der Umlaufrichtung 36 und die Halteeinrichtung 10 kann mit dem Stechmittel 5 die Stechbewegung 6 durchlaufen. Gemäß den Darstellungen der Figuren 4 und 5 ist die Stechvorrichtung 1 in einem Stechzustand dargestellt, in welchem die Halteeinrichtung 10 in ihrer translatorischen Vorwärtsbewegung 37 maximal ausgelenkt ist. Der Kurvenfolger 22 befindet sich hierbei in einer der Behandlungsöffnung 4 nächstgelegenen Position.

Vorteilhafter Weise findet bei einer translatorischen Rückwärtsbewegung 41 der Halteeinrichtung 10 gleichzeitig eine Weiterführung des Kurvenfolgers 22 in die weiterhin vorwärtsgerichtete Umlaufrichtung 36 statt, wie es insbesondere aus der Darstellung nach der Figur 4 hervorgeht. Auch diese sofortige translatorische Rückwärtsbewegung 41 wird durch die immer noch auseinanderdrückende Schenkelfeder 15 ermöglicht. Demzufolge findet nie eine wesentliche Umkehrbewegung des Kurvenfolgers 22 innerhalb der Kurvenbahn 20 des Steuerkurvenbahnelementes 13 statt. Vielmehr bewegt sich der Kurvenfolger 22 immer weiter im Uhrzeigersinn entlang der Kurvenbahn 20 bis die Stechvorrichtung 1 einen Ruhezustand in einem ungespannten Zustand erreicht, wie er gemäß der Darstellung der Figur 6 gezeigt ist.

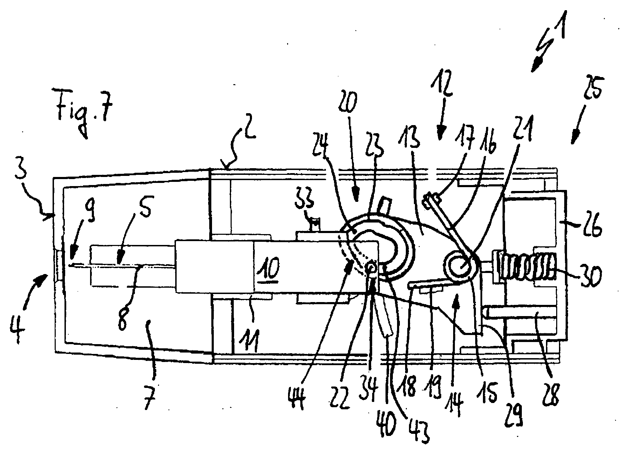

Nun kann die Stechvorrichtung 1 für eine weitere Stechbewegung 6 wieder gespannt werden, indem das handbetätigbare Tastenelement 26 in Pfeilrichtung 27 gedrückt wird, wodurch das Federelement 14 mit einer Spannbewegung 42 wieder gespannt, also zusammengedrückt, werden kann. Bei diesem Spannvorgang bewegt sich der Kurvenfolger 22 weiter entlang der Kurvenbahn 20 in Umlaufrichtung 36, bis der Kurvenfolger 22 wieder in seine Startposition 34 hinein verlagert, das Federelement 14 vorgespannt und die Stechvorrichtung 1 erneut einsatzbereit ist, wie nach der Darstellung der Figur 7 erkennbar ist. Hier sind auch ein Einlaufbereich 43 für den Kurvenfolger 22 an der Kurvenbahn 20 und ein Auslaufbereich 44 für den Kurvenfolger 22 an der Kurvenbahn 20 beziffert. Den Auslaufbereich 44 durchläuft der Kurvenfolger 22 nur beim Auslösen der Stechvorrichtung 1, wenn der Kurvenfolger 22 seine Startposition 34 verlässt. Den Einlaufbereich 43 durchläuft der Kurvenfolger 22 nur, wenn die Stechvorrichtung 1 bzw. das Federelement 14 gespannt und der Kurvenfolger 22 in die Startposition. 34 hinein bewegt wird. Insofern handelt es sich bei dem Einlaufbereich 43 und dem Auslaufbereich 44 um verschiedene Bereiche der Kurvenbahn 20.

Vorteilhafter Weise greift die Halteeinrichtung 10 bei vorliegender Stechvorrichtung 1 unmittelbar in die Kurvenbahn 20 ein, so dass eine Drehbewegung bzw. Schwenkbewegung des Steuerkurvenbahnelementes 13 direkt und besonders verlustarm in eine translatorische Stechbewegung 6 der Halteeinrichtung 10 umgewandelt werden kann.

Vorteilhaft wird vorliegend eine im Wesentlichen ovalförmige Kurvenbahn 20 hinsichtlich des Steuerkurvenbahnelementes 13 verwendet, um in den eher geraden Abschnitten der im Wesentlichen ovalförmigen Kurvenbahn 20 eine möglichst große Beschleunigung des Kurvenfolgers 22 und damit des Stechmittels 5 in Vorwärts- und anschließend in Rückwärtsrichtung zu erhalten. Dies ermöglicht idealerweise eine weitere Verminderung des Einstechschmerzes.

Die Kurvenbahn 20 dient im Wesentlichen als Leitkurve für Halteeinrichtung 10 und deren Auslösung. Somit kann das Stechmittel 5 auch lediglich nur einmal in die Haut einstechen und nicht - wie bisher bei federgeführten Systemen bekannt - zu einem Nachschwingen und somit zu einem Nachstechen bzw. mehrmaligen Einstechen in die Haut führen. Zudem weist der Gesamtaufbau der vorliegenden Stechvorrichtung 1 vorteilhaft ein besonders flaches Design auf, da alle wesentlichen beweglichen Bauteile flach ausbildet sein können und gering in ihrer Anzahl sind. Insbesondere wird nur ein Kurvenfolger 22 benötigt, der zudem in einer Kulisse 23 angeordnet ist, und der somit keine zusätzliche Bauhöhe innerhalb der Stechvorrichtung 1 erfordert.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 1: Stechvorrichtung
- 2: Gehäuse
- 3: unterseitig
- 4: Behandlungsöffnung
- 5: Stechmittel
- 6: Stechbewegung
- 7: Gehäuseinneres
- 8: Stechnadel
- 9: Stechnadelspitze
- 10: verlagerbare Halteeinrichtung
- 11: Linearführung
- 12: Mittel zum Antreiben
- 13: Steuerkurvenbahnelement
- 14: Federelement
- 15: Schenkelfeder
- 16: erster Schenkel
- 17: Gehäusehalterung
- 18: zweiter Schenkel
- 19: Halterung des Steuerkurvenbahnelementes
- 20: Kurvenbahn
- 21: Schwenkachse
- 22: Kurvenfolger
- 23: Kulisse
- 24: Endlos-Kurvenbahn
- 25: oberseitig
- 26: handbetätigbares Tastenelement
- 27: Pfeilrichtung
- 28: Tastenelementsteg
- 29: Vorsprung
- 30: Spiralfeder
- 31: Auslöseeinrichtung
- 32: Drücker
- 33: Rastarm
- 34: Startposition
- 35: Schwenkvermögen
- 36: Umlaufrichtung
- 37: translatorische Vorwärtsbewegung
- 38: Federbewegung
- 39: Führung
- 40: Bogenabschnitt
- 41: translatorischen Rückwärtsbewegung
- 42: Spannbewegung
- 43: Einlaufbereich
- 44: Auslaufbereich

## Patentansprüche

1. Stechvorrichtung (1) für eine Blutprobenentnahme mit einer verlagerbaren Halteeinrichtung (10) für ein Stechmittel (5), mit einer Linearführung (11) zum Führen der verlagerbaren Halteeinrichtung (10), mit Antriebsmitteln (12) zum Antreiben der verlagerbaren Halteeinrichtung (10) und mit einer Auslöseeinrichtung (31) zum Auslösen einer Stechbewegung (6) des Stechmittels (5), bei welcher nach einem manuellen Betätigen der Auslöseeinrichtung (31) die verlagerbare Halteeinrichtung (10) mittels der Antriebsmittel (12) translatorisch verlagert werden kann, wobei die Antriebsmittel (12) eine Kurvenbahn (20) aufweisen, in welcher die verlagerbare Halteeinrichtung (10) unmittelbar eingreift, wobei die Kurvenbahn (20) eine Endlos-Kurvenbahn (24) ist
**dadurch gekennzeichnet, dass**
die Antriebsmittel (12) ein Steuerkurvenbahnelement (13) umfassen, wobei das Steuerkurvenbahnelement (13) um eine Schwenkachse (21) schwenkbar gelagert ist, und wobei ein Kurvenfolger (22) der verlagerbaren Halteeinrichtung (10) innerhalb einer Kulisse (23) der Kurvenbahn (20) derart angeordnet ist, dass der Kurvenfolger (22) innerhalb der Kulisse (23) nur in eine einzige Umlaufrichtung (36) entlang der Kurvenbahn (20) bewegbar gelagert ist.

2. Stechvorrichtung (1) nach Anspruch 1,
**dadurch gekennzeichnet, dass**
das Steuerkurvenbahnelement (13) eine Endlos-Kurvenbahn (24) aufweist.

3. Stechvorrichtung (1) nach einem der Ansprüche 1 oder 2,
**dadurch gekennzeichnet,dass**
die Kurvenbahn (20) oval ausgebildet ist.

4. Stechvorrichtung (1) nach einem der Ansprüche 1 bis 3,
**dadurch gekennzeichnet, dass**
die Kurvenbahn (20) die Kulisse (23) aufweist, innerhalb welcher die verlagerbare Halteeinrichtung (10) zumindest teilweise angeordnet und geführt ist.

5. Stechvorrichtung (1) nach einem der Ansprüche 1 bis 4,
**dadurch gekennzeichnet, dass**
die verlagerbare Halteeinrichtung (10) einen Kurvenfolger (22) aufweist.

6. Stechvorrichtung (1) nach einem der Ansprüche 1 bis 5,
**dadurch gekennzeichnet, dass**
ein Kurvenfolger (22) der verlagerbaren Halteeinrichtung (10) innerhalb der Kurvenbahn (20) in einer Startposition (34) angeordnet ist, und die Kurvenbahn (20) hinsichtlich der Startposition (34) einen Einlaufbereich (43) für den Kurvenfolger (22) und einen Auslaufbereich (44) für den Kurvenfolger (22) aufweist, wobei der Einlaufbereich (43) und der Auslaufbereich (44) voneinander verschieden sind.

7. Stechvorrichtung (1) nach einem der Ansprüche 1 bis 6,
**dadurch gekennzeichnet, dass**
die Antriebsmittel (12) ein Federelement (14) zum Speichern von Energie umfassen.

8. Stechvorrichtung (1) nach Anspruch 7,
**dadurch gekennzeichnet, dass**
das Federelement (14) eine Schenkelfeder (15) umfasst.

9. Stechvorrichtung (1) nach Anspruch 7 oder 8,
**dadurch gekennzeichnet, dass**
das Federelement (14) an einer Schwenkachse (21) eines Steuerkurvenbahnelementes (13) gelagert ist.

10. Verfahren zum Entnehmen einer Blutprobe, bei welchem ein Stechmittel (5) vorwärts und anschließend direkt rückwärts bewegt wird, wodurch das Stechmittel (5) zumindest mit seiner Spitze (9) kurzzeitig durch eine Behandlungsöffnung (4) kurzzeitig aus einem Gehäuseinneren (7) der Stechvorrichtung (1) hervorschnellen kann, bei welchem das Stechmittel (5) in einer verlagerbaren Halteeinrichtung (10) translatorisch geführt wird, und bei welchem die verlagerbare Halteeinrichtung (10) von einem Federantrieb angetrieben wird, wobei die verlagerbare Halteeinrichtung (10) unmittelbar innerhalb und entlang einer Endlos-Kurvenbahn (24) eines Steuerkurvenbahnelementes (13) geführt wird,
**dadurch gekennzeichnet, dass**
das Steuerkurvenbahnelement (13) um eine Schwenkachse (21) schwenkbar gelagert ist, wobei ein Kurvenfolger (22) der verlagerbaren Halteeinrichtung (10) innerhalb einer Kulisse (23) einer Kurvenbahn (20) derart angeordnet ist, dass der Kurvenfolger (22) innerhalb der Kulisse (23) nur in eine einzige Umlaufrichtung (36) entlang der Kurvenbahn (20) bewegbar gelagert ist.

11. Verfahren nach Anspruch 10,
**dadurch gekennzeichnet, dass**
die verlagerbare Halteeinrichtung (10) sowohl bei einer translatorischen Vorwärtsbewegung (37) des Stechmittels (5) als auch bei einer translatorischen Rückwärtsbewegung (41) des Stechmittels (5) immer nur in eine einzige Umlaufrichtung (36) entlang der Endlos-Kurvenbahn (24) bewegt wird.

12. Verfahren nach Anspruch 10 oder 11,
**dadurch gekennzeichnet, dass**
die Endlos-Kurvenbahn (24) entlang eines Bogenabschnittes (40) um eine Schwenkachse (21) geschwenkt wird, wodurch die verlagerbare Halteeinrichtung (10) translatorisch bewegt wird.

## Claims

1. Pricking device (1) for taking a blood sample, comprising a displaceable retaining device (10) for a pricking means (5), comprising a linear guide (11) for guiding the displaceable retaining device (10), comprising drive means (12) for driving the displaceable retaining device (10), and comprising a trigger device (31) for triggering a pricking movement (6) of the pricking means (5), in which, after manual actuation of the trigger device (31), the displaceable retaining device (10) can be displaced in translation by the drive means (12), the drive means (12) having a curved track (20) in which the displaceable retaining device (10) engages directly, the curved track (20) being an endless curved track (24), **characterised in that** the drive means (12) comprise a control curved track element (13), the control curved track element (13) being pivotally mounted about a swivel axis (21), and a curved follower (22) of the displaceable retaining device (10) being arranged inside a slotted link (23) of the curved track (20) such that the curved follower (22) is mounted inside the slotted link (23) so as to be movable along the curved track (20) in just one peripheral direction (36).

2. Pricking device (1) according to claim 1, **characterised in that** the control curved track element (13) has an endless curved track (24).

3. Pricking device (1) according to either claim 1 or claim 2, **characterised in that** the curved track (20) is oval.

4. Pricking device (1) according to any of claims 1 to 3, **characterised in that** the curved track (20) comprises the slotted link (23) inside which the displaceable retaining device (10) is arranged and guided at least in part.

5. Pricking device (1) according to any of claims 1 to 4, **characterised in that** the displaceable retaining device (10) has a curved follower (22).

6. Pricking device (1) according to any of claims 1 to 5, **characterised in that** a curved follower (22) of the displaceable retaining device (10) is arranged inside the curved track (20) in a starting position (34), and, with regard to the starting position (34), the curved track (20) has an inlet region (43) for the curved follower (22) and an outlet region (44) for the curved follower (22), the inlet region (43) and the outlet region (44) being different from each other.

7. Pricking device (1) according to any of claims 1 to 6, **characterised in that** the drive means (12) comprise a spring element (14) for storage of energy.

8. Pricking device (1) according to claim 7, **characterised in that** the spring element (14) comprises a leg spring (15).

9. Pricking device (1) according to either claim 7 or claim 8, **characterised in that** the spring element (14) is mounted on a swivel axis (21) of a control curved track element (13).

10. Method for taking a blood sample in which a pricking means (5) is moved forward and then directly backward, as a result of which at least the tip (9) of the pricking means (5) can briefly shoot through a treatment opening (4), out of a housing interior (7) of the pricking device (1), in which the pricking means (5) is guided in translation in a displaceable retaining device (10), and in which the displaceable retaining device (10) is driven by a spring drive, the displaceable retaining device (10) being guided directly inside and along an endless curved track (24) of a control curved track element (13), **characterised in that** the control curved track element (13) is pivotally mounted about a swivel axis (21), a curved follower (22) of the movable retaining device (10) being arranged inside a slotted link (23) of a curved track (20) such that the curved follower (22) is mounted inside the slotted link (23) so as to be movable along the curved track (20) in just one peripheral direction (36).

11. Method according to claim 10, **characterised in that** the displaceable retaining device (10) is always moved in just one peripheral direction (36) along an endless curved track (24), both during a translational forward movement (37) of the pricking means (5) and during a translational reverse movement (41) of the pricking means (5).

12. Method according to either claim 10 or claim 11, **characterised in that** the endless curved track (24) is pivoted along an arc portion (40) about a swivel axis (21), as a result of which the displaceable retaining device (10) is moved in translation.

## Revendications

1. Dispositif de piqûre (1) pour un prélèvement d'échantillon de sang, avec un dispositif de maintien mobile (10) pour un moyen de piqûre (5), avec un guidage linéaire (11) pour le guidage du dispositif de maintien mobile (10), avec des moyens d'entraînements (12) pour l'entraînement du dispositif de maintien mobile (10) et avec un dispositif de déclenchement (31) pour le déclenchement d'un mouvement de piqûre (6) du moyen de piqûre (5), dans lequel, après un actionnement manuel du dispositif de déclenchement (31), le dispositif de maintien mobile (10) peut être déplacé en translation à l'aide des moyens d'entraînement (12), les moyens d'entraînement (12) comprenant une piste courbe (20), dans laquelle le dispositif de maintien mobile (10) s'emboîte directement, la piste courbe (20) étant une piste courbe sans fin (24),
**caractérisé en ce que**
les moyens d'entraînement (12) comprennent un élément de commande à piste courbe (13), l'élément de commande à piste courbe (13) étant logé de manière pivotante autour d'un axe de pivotement (21) et un galet suiveur (22) du dispositif de maintien mobile (10) étant disposé à l'intérieur d'une coulisse (23) de la piste courbe (20) de façon à ce que le galet suiveur (22) soit logé de manière mobile à l'intérieur de la coulisse (23) dans un seul sens de rotation (36) le long de la piste courbe (20).

2. Dispositif de piqûre (1) selon la revendication 1,
**caractérisé en ce que**
l'élément de commande à piste courbe (13) comprend une piste courbe sans fin (24).

3. Dispositif de piqûre (1) selon l'une des revendications 1 ou 2,
**caractérisé en ce que**
la piste courbe (20) présente une forme ovale.

4. Dispositif de piqûre (1) selon l'une des revendications 1 à 3,
**caractérisé en ce que**
la piste courbe (20) comprend la coulisse (23), à l'intérieur de laquelle le dispositif de maintien mobile (10) est au moins partiellement disposé et guidé.

5. Dispositif de piqûre (1) selon l'une des revendications 1 à 4,
**caractérisé en ce que**
le dispositif de maintien mobile (10) comprend un galet suiveur (22).

6. Dispositif de piqûre (1) selon l'une des revendications 1 à 5,
**caractérisé en ce qu'**
un galet suiveur (22) du dispositif de maintien mobile (10) est disposé à l'intérieur de la piste courbe (20) dans une position de départ (34) et la piste courbe (20) comprend, en ce qui concerne la position de départ (34), une zone d'entrée (43) pour le galet suiveur (22) et une zone de sortie (44) pour le galet suiveur (22), la zone d'entrée (43) et la zone de sortie (44) étant distinctes.

7. Dispositif de piqûre (1) selon l'une des revendications 1 à 6,
**caractérisé en ce que**
les moyens d'entraînement (12) comprennent un élément à ressort (14) pour le stockage d'énergie.

8. Dispositif de piqûre selon la revendication 7,
**caractérisé en ce que**
l'élément à ressort (14) comprend un ressort à branches (15).

9. Dispositif de piqûre selon la revendication 7 ou 8,
**caractérisé en ce que**
l'élément à ressort (14) est logé au niveau d'un axe de pivotement (21) d'un élément de commande à piste courbe (13).

10. Procédé de prélèvement d'un échantillon de sang, dans lequel un moyen de piqûre (5) est déplacé vers l'avant puis directement vers l'arrière, le moyen de piqûre (5) pouvant ainsi surgir, au moins avec sa pointe (9), brièvement à travers une ouverture de traitement (4), brièvement hors de l'intérieur d'un boîtier (7) du dispositif de piqûre (1), dans lequel le moyen de piqûre (5) est guidé en translation dans un dispositif de maintien mobile (10) et dans lequel le dispositif de maintien mobile (10) est entraîné par un entraînement à ressort, le dispositif de maintien mobile (10) étant guidé directement à l'intérieur et le long d'une piste courbe sans fin (24) d'un élément de commande à piste courbe (13),
**caractérisé en ce que**
l'élément de commande à piste courbe (13) est logé de manière pivotante autour d'un axe de pivotement (21), un galet suiveur (22) du dispositif de maintien mobile (10) est disposé à l'intérieur d'une coulisse (23) d'une piste courbe (20) de façon à ce que le galet suiveur (22) soit logé de manière mobile à l'intérieur de la coulisse (23) dans un seul sens de rotation (36) le long de la piste courbe (20).

11. Procédé selon la revendication 10,
**caractérisé en ce que**
le dispositif de maintien mobile (10) est déplacé aussi bien lors d'un mouvement vers l'avant en translation (37) du moyen de piqûre (5) que lors d'un mouvement vers l'arrière en translation (41) du moyen de piqûre (5), dans un seul sens de rotation (36) le long de la piste courbe sans fin (24).

12. Procédé selon la revendication 10 ou 11,
**caractérisé en ce que**
la piste courbe sans fin (24) est pivotée le long d'une portion d'arc (40) autour d'un axe de pivotement (21), ce qui permet de déplacer le dispositif de maintien mobile (10) en translation.
